# EUROPEAN PATENT APPLICATION

(11) **EP 1 473 034 A1**
(43) Date of publication of application: **03.11.2004**
(21) Application number: 03005488.6
(22) Date of filing: 17.03.2003
(51) Int. Cl.: A61K 31/4015, A61K 31/522, A61K 31/277, A61K 31/44, A61K 31/421, A61K 31/5513, A61K 31/52, A61K 31/404, A61K 31/343, A61K 31/4709, A61K 31/4166, A61K 38/16, A61K 38/00, A61K 31/4409, A61P 15/08

(54) **Use of an inhibitor of the phosphodiesterase-4-enzyme (PDE4) alone or in combination with relaxin and derivatives thereof for the manufacture of a medicament to promote decidualization of endometrial cells and to increase fertility**

(71) Applicant: Lagow GmbH, 22529 Hamburg (DE)
(72) Inventor: Ivell, Prof. Dr. Richard, 25451 Qickborn (DE); Bartsch, Dr. Olaf, 22523 Hamburg (DE)
(74) Representative: UEXKÜLL & STOLBERG

(57) **Abstract**

This invention relates to the use of an inhibitor of the phosphodiesterase-4 enzyme (PDE4) for the preparation of a pharmaceutical composition for promoting the decidualization of endometrial cells.

The pharmaceutical composition may further comprise a compound which increases the synthesis of cAMP, such as relaxin or an agonist of the action of relaxin receptors such as relaxin receptors LGR7 and/or LGR8.

## Description

This invention relates to the use of an inhibitor of the phosphodiesterase-4 enzyme (PDE4) for the preparation of a pharmaceutical composition for promoting the decidualization of endometrial cells. The pharmaceutical composition may further comprise an agonist of the action of relaxin receptors.

During the menstrual cycle, the stromal cells of the endometrium undergo a progressive and controlled differentiation process to meet the evolutionary demands of providing an adequate maternal interface for the implanting blastocyst. Prior to ovulation, the endometrium is in a proliferative phase, with few relatively underdeveloped glands and spiral arteries. This phase is typified by stromal cells of a relatively undifferentiated mesenchymal phenotype. Following ovulation, when the concentration of circulating progesterone increases, the stromal cells of the proliferative endometrium differentiate, changing shape to a more polygonal phenotype. This process of stromal cell decidualization is accompanied by a marked development of both endometrial glands and spiral arteries. Adequate decidualization is essential for the endometrium to be able to support implantation of a newly formed embryo.

At a molecular level, decidualization is characterized by the upregulation of genes such as those for prolactin [Telgmann R., Gellersen B., Hum. Reprod. Update **4**: 472-479 (1998)], IGF-BP1 [Irwin J.C. et al., Regul. Pept. **48**: 165-177 (1993)], or glycodelin [Seppala M. et al., Hum. Reprod. **9**: 917-925 (1994); Stewart D.R. et al., J. Clin. Endocrinol. Metab. **82**: 839-846 (1997)], which are among the major secretory proteins of the human decidualized endometrium during pregnancy. Additional factors are secreted *in vivo,* which are necessary for correct epithelial differentiation, such that the latter cells can properly accommodate an implanting blastocyst in the so-called "window of implantation" [Wilcox A.J. et al., N. Engl. J. Med. 340: 1796-1799 (1999); Paria B.C. et al. Science **296**: 2185-2188 (2002); Lindhard A. et al, Fertil. Steril. **78:** 221-233 (2002)].

Two out of three blastocysts fail to implant in otherwise healthy women. In subfertile and infertile women, this proportion is considerably higher. While part of this implantation failure can be attributed to poor embryo quality, there may also be a marked endometrial deficit in the capacity of the endometrium to support implantation. Endometrial development is known to be important for in vitro fertilization outcome [Gonen Y., Casper R.F., J. In Vitro Fert. Embryo Transf. **7**: 146-152 (1990); Sher G. et al., Hum. Reprod. **6**: 232-237 (1991); Noyes N. et al., Hum. Reprod. **10**: 919-922 (1995)], and supplementary hormonal (luteal) support has been recommended in order to improve the receptivity of the endometrium [Soliman S. et al., Fertil. Steril. **61**: 1068-1076 (1994); Pritts E.A., Atwood A.K., Hum. Reprod. **17**: 2287-2299 (2002)]. Furthermore, disruption of endometrial differentiation is associated with several gynecological disorders such as endometriosis, adenomyosis and luteal phase defect, and is an important cause of infertility and menstrual disorders [e.g.

Daley D.C. et al., Am. J. Obstet. Gynecol. **140**: 587-591 (1981); Bulun S.E. et al., J. Steroid Biochem. Mol. Biol. **61**: 133-139 (1997); Brosens J., Brosens I., in Cameron I. et al. (eds.) "Clinical disorders of the menstrual cycle and endometrium." Oxford University Press, New York: 297-312 (1998)].

Whilest the importance of the steroid hormones estradiol and progesterone is unquestioned in vivo, their precise mechanism of action in the endometrium is not known. At a cell biological level, decidualization of endometrial stromal cells can be induced in vitro by a high concentration of progesterone, following estradiol-priming [Huang J.R. et al., Endocrinology **121**: 2011-2017 (1987)], as well as by a variety of compounds which have in common the ability to increase the intracellular concentration of cAMP. These compounds include natural hormones such as relaxin [Huang J.R. et al. supra; Tabanelli S. et al., J. Steroid Biochem. Mol. Biol. **42**: 337-344 (1992)], prostaglandin E2 or gonadotropins, such as hCG or LH [Tang B., Gurpide E., J. Steroid Biochem. Mol. Biol. **47**: 115-121 (1993)], as well as reagents like 8Br-cAMP [Tang B. et al., Endocrinology **133**: 2197-2203 (1993)] or the adenylate cyclase activator forskolin [Tang B. et al., *supra].*

Intracellular cAMP concentrations are the result of an interplay between cAMP generation and hydrolysis by specific phosphodiesterases (PDE). The latter provide a powerful means of manipulating the magnitude and duration of the biological response to cAMP. It was shown previously that the ability of hormones, such as relaxin, to induce the production of cAMP, can be enhanced by the addition of general inhibitors of phosphodiesterase (PDE) activity, such as 3-isobutyl-1-methylxanthine (IBMX) (Bartsch O. et al., Mol. Hum. Reprod. 7: 799-809 (2001)].

Indeed, recent experiments have suggested that relaxin is most probably acting through the newly discovered 7TM-receptor, LGR7 [Hsu et al., 2002 Activation of orphan receptors by the hormone relaxin. Science 295:671-674], which when overexpressed in transfected cells appears to couple to adenylate cyclase via Gs [Hsu et al., 2002, supra]. Further, relaxin may even be signalling through a regulation of endogenous PDE activity [Bartsch O. et al. (2001) *supra;* Bartsch O. et al. in Tregear G.W. et al. (eds.) "Relaxin 2000", Kluwer Academic Publishers, Netherlands: 309-315 (2001)].

It has recently been suggested that the endogenous relaxin receptor couples only poorly to adenylate cyclase via Gs [Kompa et al., 2002, Br J Pharmacol 137:710-718], and that an alternative, tyrosine kinase-dependent pathway may link receptor activation to a still uncharacterized PDE [Bartsch et al., 2001, supra]. This is based on the ability of a specific group of tyrphostins, usually considered specific for the EGF-receptor tyrosine kinase or the PDGF-receptor tyrosine kinase to completely inhibit relaxin-induced cAMP production in human endometrial stromal cells and in THP1 cells [Bartsch et al., 2001, supra]. Furthermore, unlike conventional Gs-coupled receptors such as VIP, relaxin cannot induce adenylate cyclase activation in membrane preparations of endometrial stromal cells [Bartsch et al., 2001, supra]. Finally, relaxin-induced cAMP production can be inhibited by the MAP kinase inhibitors PD98059 and U0126 [Bartsch et al., 2001, Academic Publishers, Netherlands :309-315]. All of these points suggest that activation of endogenous LGR7 is atypical, with only weak coupling to adenylate cyclase, and that another pathway, probably using a MAP kinase cascade is involved.

Endometrial development is still poorly understood, although pharmaceutical compositions which promote endometrial development could be used to improve fertility in healthy and infertile women. The problem underlying the present invention thus resides in the identification of compounds, which promote endometrial development.

This problem is now solved by the use of an inhibitor, which specifically inhibits the phosphodiesterase-4 enzyme (PDE4), for the preparation of a pharmaceutical composition for promoting the decidualization of endometrial cells. According to a preferred embodiment, the use comprises the the preparation of a pharmaceutical composition for increasing the rate of pregnancy.

The present inventors have surprisingly shown that PDE4 specific inhibitors can be used to promote decidualization of the endometrial cells. Sofar inhibitors of PDE4 were merely suggested as targets for treating entirely unrelated diseases, including heart failure, depression, rheumatoid arthritis, atopic dermatitis, asthma and myometrial quiescence [Stafford and Feldmann, 1996, Annual reports in Medicinal Chemistry (Doherty, SM Ed.):71-80, Academic Press, San Diego, Ca, USA; Teixera et al., 1997; Méhats et al. 2000, Endocrinology 140:3228-3237; Dyke et al., 2002, Expert Opin Investig Drugs 11:1-13]. The present inventors were able to show that a PDE4 specific inhibitor (such as rolipram alone) is able to induce decidualization-specific genes, like prolactin and IGF-BP1, secretion of prolactin peptide, and appropriate morphological appearance (Christian et al., Reprod Biomed Online, 3:24-30, 2002). The inhibitor is thus specific for PDE4 and does not inhibit all PDEs unspecifically. The use of a PDE4 specific inhibitor has the advantage that less side effects will be caused by the treatment.

Decidualization of endometrial stromal cells in the secretory phase of the menstrual cycle is an essential prerequisite for adequate implantation of a blastocyst. Promotion of decidualization helps to encourage the blastocyst's implantation into the endometrium. Promotion of decidualization supports the early growth of both the embryo and the trophoblast and is therefore beneficial both to female fertility and embryonal development, which leads to reduction of the risk of an early loss of the embryo during the first trimester of pregnancy. Application of a PDE4 inhibitor is suitable to promote decidualization and endometrial development.

Decidualization is defined as the profound differentiation process of the uterine endometrium in the late luteal phase of the menstrual cycle in order to accomodate the implanting embryo. In the proliferative or follicular phase intensive proliferation leads to a thickening of the endometrium from 0.5mm to 2-3mm and to the developement of epithelial glands (Fawcett, A Textbook of Histology. Chapman & Hall, New York, 1994).

In the secretory or luteal phase the decidualized endometrium achieves its maximal thickness of 4-6mm. The intensive secretory differentiation is accompanied by the developement of spiral arteries up to the surface of the luminal epithel and by wavy lined glands with a wide lumen (Fawcett, 1994).

In the late luteal phase, decidualization is typified by additional markers as reported by Noyes et al. (Fertil Steril 1: 3-25, 1950) and Giudice & Ferency (The Endometrial Cycle. In: Reproductive Endocrinology, Surgery, and Technology (eds. Adashi EY, Rock JA, Rosenwaks Z) Lippincott-Raven, Philadelphia, New York, 271-3001996).

In vivo the thickness of the endometrium is preferentially measured by sonography and the morphological appearance of biopsies are analysed under the microscope. Moreover, a trilaminar sonographic endometrial pattern of > 8mm on the day of human chorionic gonadotropin (HCG) has been shown to be correlated with high chance of pregnancy in patients treated with IVF (Gonen et al., J In Vitro Fertil Embryo Transfer 7: 146-152, 1990; Sher et al., Hum Reprod 6: 232-237, 1991). Based on our initial investigation on the positive effect of PDE4 inhibition on in vitro decidualization, we propose the treatment of women with a PDE4 inhibitor in order to improve endometrial developement and decidualization measured as described above. The benefits of PDE4 inhibition should be evaluated in randomized clinical trials.

Decidualization is analysed in vitro using a cell culture system of primary endometrial stromal cells in which endometrial tissue from hysterectomies of cycling women was digested enzymatically to isolate endometrial stromal cells. Decidualization is induced in vitro by contacting the endometrial stromal cells in cell culture with a PDE4 specific inhibitor, preferentially in combination with a cAMP-elevating agent like relaxin. A promotion of decidualization is observed, when endometrial stromal cells in culture show a statistically significant increase in the expression of decidual markers such as the decidual marker genes prolactin or IGF-BP1 or secretion of prolactin or IGF-BP1 (Telgmann & Gellersen, 1998; Irwin et al., 1993).

In accordance with the present invention a compound is referred to a as cAMF specific PDE inhibitor, if it inhibits the activity of PDE4 in vitro using the phosphodiesterase assay as described in Hansen and Beavo (Proc Natl Acad Sci USA, 79:2788-2792, 1982).

Additionally, decidualization can be observed under the microscope, when the endometrial stromal cell has differentiated from an elongated phenotype to the typical larger, rounder phenotype of the decidual cell (Christian et al., Reprod Biomed Online 4:24-30, 2002).

According to one aspect of the present invention the PDE4 inhibitor has an inhibiting effect on one or several of the different PDE4 isoforms. PDE4 activity is encoded by four related genes (PDE4A, -4B, -4C and -4D) which encode a large number of isoforms generated via alternate splicing of RNA [Houslay et al., 1998, Adv Pharmacol 44:225-342]. Inhibitors with inhibiting activity on all isoforms are preferably used for the preparation of the pharmaceutical composition.

In a preferred embodiment the inhibitor is rolipram (Schering/Calbiochem), Ro-20-1724 (Calbiochem), Arophyline (Allmiral), Cilomilast (GlaxoSmithKline), Roflumilast (Byk Gulden), V-11294A (Napp), CDC-801 (Celgene), Bay-19-8004 (Bayer), Cipamphylline (SmithKlineBeecham), SCH-351591 (Schering Plough), D-4418 (Schering Plough), YM-976 (Yamanouchi), PD-189659 (Pfizer), CI-1018.9 (Park Davis), Mesopram (Schering AG), Pumafentrine (Byk Gulden), CDC-998 (Celgene), IC-485 (Icos), KW-4490 (Kyowa), RPR-73401, RPR-109026, L-791,943 (Merck), CDP-840 (Celltech), CI-1044, XT-44 or pentoxyfylline.

The pharmaceutical composition may contain a single inhibitor or a mixture of inhibitors.

In a preferred embodiment of the present invention, the pharmaceutical composition for promoting the decidualization of endometrial cells comprises an inhibitor, which specifically inhibits the phosphodiesterase-4 enzyme (PDE4), and further a compound which increases the intracellular synthesis of cAMP. The compound can for example be relaxin itself or an agonist of the action of a relaxin receptor. In accordance with the present invention a compound is considered to be an agonist of the relaxin receptor, if the compound leads to an increase in intracellular cAMP upon binding of the relaxin receptor. The relaxin receptor can for example be the relaxin receptor LGR7 and/or LGR8.

In the pesent application a compound is an agonist of the action of a relaxin receptor if it binds to the receptors LGR7 and/or LGR8 and stimulates cAMP production as described in Hsu et al. (Science, 295, 671-674, 2002).

According to an alternative embodiment the compound which increases the synthesis of cAMP is a peptide analogue or a non-peptide mimetic.

In a further embodiment, the present invention is direceted to pharmaceutical compositions comprising an inhibitor, which specifically inhibits the phosphodiesterase-4 enzyme (PDE4), and relaxin or an agonist of the action of relaxin receptors.

The agonist of relaxin of the action of relaxin receptors can be a peptide analogue or a non-peptide mimetic.

The PDE4 inhibitor in the pharmaceutical composition can have an inhibiting effect on one or several of the PDE4 isoforms, i.e. PDE4-A, -B, -C, -D. Preferably, the pharmaceutical composition comprises one of the following inhibitors is rolipram (Schering/Calbiochem), Ro-20-1724 (Calbiochem), Arophyline (Allmiral), Cilomilast (GlaxoSmithKline), Roflumilast (Byk Gulden), V-11294A (Napp), CDC-801 (Celgene), Bay-19-8004 (Bayer), Cipamphylline (SmithKlineBeecham), SCH-351591 (Schering Plough), D-4418 (Schering Plough), YM-976 (Yamanouchi), PD-189659 (Pfizer), CI-1018.9 (Park Davis), Mesopram (Schering AG), Pumafentrine (Byk Gulden), CDC-998 (Celgene), IC-485 (Icos), KW-4490 (Kyowa), RPR-73401, RPR-109026, L-791,943 (Merck), CDP-840 (Celltech), CI-1044, XT-44 or pentoxyfylline.

The pharmaceutical composition for promoting the decidualization of endometrial cells containing an inhibitor of the phosphodiesterase-4 enzyme (PDE4) alone or a combination therof with an agonist of the action of relaxin receptors is preferably used for the treatment of humans, specifically for the treatment of women.

Promotion of the decidualization using the pharmaceutical composition of the present invention will increase the blastocyst implantation rate and thus increase the rate of pregnancy. For this purpose, the pharmaceutical compostion is preferably administered before and/or during the first trimester of pregnancy.

The medical use of the present invention can be used to increase the fertility rate in healthy and infertile women. According to an especially preferred embodiment the implantation rate of the blastocyst after artificial fertilization is supported by amdinistration of the pharmaceutical composition before and/or after implantation of the blastocyst. According to one aspect of the invention, the fertilization is in vitro-fertilization.

The pharmaceutical composition of the present invention may alternatively be used for the treatment of endometrial disorders, such as endometriosis, adenomyosis, luteal phase defects and menstrual disorders.

In accordane with the present invention the pharamceutical composition comprises an inhibitor of the phosphodiesterase-4 enzyme (PDE4) for promoting the decidualization of endometrial cells and optionally a compound, which increases the intracellular synthesis of cAMP, such as relaxin or an agonist of the action of relaxin receptors, and a pharmaceutically acceptable carrier and/or diluent.

The pharmaceutical composition can be formulated for any suitable administration route. Oral, intravenous or vaginal administration is preferred. The concentration of the PDE4 inhibitor in the pharmaceutical composition can be determined by one of ordinary skill in the art using standard assays.

In one aspect of the present invention the pharmaceutical composition comprising the PDE4 specific inhibitor alone or in combination with relaxin or an agonist of the action of relaxin receptors is administered repeatedly over a period of at least 3 days, preferably over a period of at least 2 weeks. Depending on the form of administration and the formulation of the composition the same can be administered dayly or several times per day.

According to a specifically preferred embodiment, the pharmaceutical composition comprising the PDE4 specific inhibitor in combination with relaxin is administered over a period of at least 3 days several times a day to women before and/or after implantation of the blastocyst.

The following Examples illustrate the surprising activity of PDE4 inhibitors alone and in combination with relaxin. All experiments were performed at least in triplicate and, where appropriate, data were compared by ANOVA, followed by a post hoc Student-Newman-Keuls test. Differences were considered statistically significant, when P < 0.05. The FIAs and ELISAs used in these examples are described in detail therein. Alternatively one could use commercially available kits from any of the following sources:

| | |
|---|---|
| cAMP-ELISA | IBL Hamburg GmbH R&D Systems; Minneapolis Molecular Devices GmbH; München |
| | |
| Prolaktin-ELISA (human) | IBL Hamburg GmbH Bioserv Diagnostics; Rostock MD Biosciences, Canada Bio Quant, San Diego KMI-Diagnostics, Minneapolis Yes Biotech Laboratories Ltd., Canada |

### BRIEF DESCRIPTION OF THE FIGURES

### Figure 1

Phosphodiesterase activity, measured as cAMP degraded, in extracts of ESC cells in the absence or presence of specific phosphodiesterase inhibitors (10*µ*M) as indicated.

### Figure 2

Intracellular cAMP in ESC cells stimulated (+) or not (-) with the hormone relaxin, in the absence or presence of various PDE inhibitors, as indicated, applied at 100*µ*M or 1mM concentrations.

### Figure 3

RT-PCR reactions specific for PDE4A-4D transcripts (A) and PDE8 transcripts (C). PCR products were densitometrically quantified from four independent (different cell batches) experiments (B). GAPD mRNA was measured as control. In panel C, PDE8 transcripts showed no variation under cAMP stimulation (not shown); RT-PCR products from ESC cells are compared with those from the THP-1 human monocyte cell line. c indicates control reactions where the reverse transcriptase step has been omitted.

### Figure 4

Effect of the protein kinase A inhibitor H89 on relaxin-dependent (+) stimulation of cAMP production in ESC cells, in the presence and absence of the specific PDE4 inhibitor rolipram. Note, that while rolipram shows a considerable synergy with relaxin in the controls, there is almost no effect on this of the PKA inhibitor, H89.

### Figure 5

RT-PCR analysis for prolactin (PRL) and insulin-like growth factor binding protein-1 (IGF-BP1) transcripts in ESC cells cultured for 3 days in the absence or presence of progesterone, relaxin and/or the PDE inhibitors papaverine and rolipram. 8Br-cAMP was used as positive control. **A.** PCR products from a typical experiment; GAPD mRNA was assessed as control for RNA integrity. **B.** Densitometric quantification from 4 independent experiments (different cell batches).

### Figure 6

Prolactin production measured by specific ELISA in the media of ESC cells cultured for 6 days in the absence or presence of progesterone (P), relaxin (RLX) and/or the PDE inhibitors rolipram and papaverine (PAP). Essentially similar results were evident already after 3 days of culture (not shown). 8Br-cAMP was applied as positive control.

### Figure 7

Morphology of human ESC cells cultured in the absence or presence of relaxin (RLX) and/or the PDE4 inhibitor rolipram for 2 days. At this time point the cultures show maximum divergence in the progression of in vitro decidualization, as indicated by the typical polygonal cobblestone appearance of the cells. Unstimulated cells retain their relatively undifferentiated fusiform shape (upper panel). 8Br-cAMP was used as positive control.

### Example 1

### Cell Culture and Drug Treatments.

All patient samples were collected in accord with the Helsinki declaration and the authorization of the local ethical commitee. Human endometrial stromal cells (ESC) were prepared from uterine samples of premenopausal and cycling woman (35-50 years old), undergoing hysterectomy for leiomyoma. Routinely, primary cultures of >95% purity were obtained and cultured using established methods (Gellersen et al. 1994; Bartsch et al., 2001a). Each experiment used tissue from a single individual. Human ESC cells were cultured in T80 flasks (Nunc, Roskilde, Denmark) in basal medium containing DMEM-Ham's F-12 at a 1:1 ratio, 10% fetal calf serum (FCS) that had been depleted of steroids by treatment with dextran-coated charcoal, 100 IU/ml penicellin, 100*µ*g/ml streptomycin, 1*µ*g/ml insulin and 10⁻⁹M 17β-oestradiol (Sigma, Deisenhofen, Germany). Cells were taken between passage 2 and passage 6, and finally transferred to 6-well and 12-well plates (Nunc) or to 92X17 TC dishes (Nunc), as indicated. Progesterone (Sigma) and/or relaxin (>85% pure porcine relaxin, giving a single Coomassie stained band in PAGE; courtesy Dr O.D. Sherwood) were added to the medium, where indicated, at concentrations of 250nM and 100ng/ml, respectively. Previous studies had shown the EC₅₀ for this porcine relaxin in ESC to be ~1.4nM (equivalent to 10ng/ml porcine relaxin) [Bartsch et al., 2001a].

Acute effects of phosphodiesterase inhibition on intracellular cAMP concentration, following a 20min exposure to relaxin, were measured using a cAMP fluorometric immunoassay (FIA; see below). Confluent cultures of ESC in 12-well plates were preincubated for 30min with 100*µ*M of the PDE4 selective inhibitors rolipram or Ro-20-1724 (Calbiochem, La Jolla, CA) or with 100*µ*M and 1mM of the non-selective PDE inhibitors IBMX (Calbiochem) or papaverine (Sigma). Additionally, ESC cultures were pretreated with 10*µ*M of the protein kinase A (PKA) inhibitor H89 (Calbiochem) for 60min, prior to incubation with PDE-inhibitors and/or relaxin.

Longer term studies on the effect of phosphodiesterase-inhibition on the *in vitro* decidualization of ESC were perfomed in 6-well plates by coincubating relaxin and/or progesterone with 100*µ*M final concentrations of either rolipram or papaverine, using incubation times from 48-144 h, as indicated. Alternatively, 8Br-cAMP (1mM: BioLog, Bremen, Germany) was added to the medium as positive control. Medium was changed every 48hrs. Cells were either harvested for RNA preparation (see below) or conditioned medium was collected and stored at -80°C, until measurement of secreted prolactin (see below). All PDE Inhibitors were dissolved in dimethyl sulfoxide (DMSO) and supplemented to cultures to give a final concentration of 0.1% DMSO, which was used as negative control in all experiments. The human monocyte cell-line THP-1 was purchased from the European Cell Culture Collection (ECACC, no. 88081201) and cultured as described previously [Bartsch et al., 2001, supra].

### Example 2

### The Effect of PDEs on cAMP Concentration

### cAMP Determination

ESC cells in 12-well plates were incubated with combinations of relaxin and different inhibitors of phosphodiesterase as described before. For measurement of intracellular cAMP, confluent monolayers were washed twice with ice-cold PBS and extracted in 1ml cold ethanol at -20°C overnight. cAMP content was measured using a conventional enzyme-linked immunosorbent assay (ELISA) as described previously (Zarreh-Hoshyari-Khah et al., 2001, Regul Pept 97:139-146), or a modification of this assay using time-resolved fluorescence (FIA), as follows. After centrifugation of any cell debris, cell-lysates in 70% ethanol were evaporated to dryness, taken up in E-PBS buffer (0.1M sodium phophate, pH7.0; 0,.15M NaCl; 5mM EDTA; 0.2% bovine serum albumin; 0.01% thimerosal), and acetylated with 0.05% (v/v, final concentration) of freshly prepared acetylation mixture (1 vol. acetic anhydride plus 2 vol. triethylamin. Acetylated cAMP was then measured by FIA, with a europium (Eu³⁺) chelate-labeled cAMP-derivative (cAMP-DPA-Eu³⁺) as tracer, competing with the cAMP in the sample for binding to a cAMP-specific rabbit polyclonal antibody. For detection, Eu³⁺ was dissociated from the tracer by incubation with a commercially available enhancement solution (Wallac #1244-105, Perkin Elmer Life Sciences, Boston, USA) rapidly to form a new highly fluorescent complex. Finally, samples were pulsed with excitation light of 340nm and emitted light was detected at 615nm in a Wallac Victor² 1420 multilabel counter (Perkin Elmer Life Sciences). Full protocoll details are as follows:

### cAMP-FIA

### Materials:

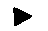 Microtiter strips: FluoroNunc Module (incl.frame); Nunc 437915, coated with goat anti-rabbit-gamma-globulin
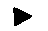 Lid: (Nunc 263339)
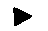 Assay buffers (EPBS):
   0.10M sodium phosphate
   0.15M NaCl
   0.005M EDTA
   0.20% BSA
   0.01% Thimerosal
   pH 7.0
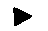 cAMP-antiserum: affinity purified antiserum (MS1 rabbit). Pool 6-25, dilution 1:300 in assay buffer + 0.0005% methylorange
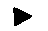 cAMP-DTPA-Eu3+ (tracer):stock : lyophilized aliquots ready-to-use : ca.50.000 cps / 50*µ*l dissolve lyophilizate in tracer buffer
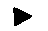 tracer buffer:
   0.1M Tris/HCl
   0.1% NaN3
   0.9% NaCl
   0.01% Tween20
   0.1% BSA
   pH 7.5
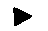 wash solution: 0.02% Tween20 in H2O 0.5% NaCl
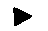 enhancement solution:
   15uM naphtoyltrifluoroacetone (NTA)
   50uM triocytylphosphinoxid (TOPO)
   0.1M acetic acid
   0.1% Triton X-100
   potassium hydrogen phthalate to pH 3.2
   commercial solution Wallac 1244-105

### Working protokoll for cAMP-FIA:

Plate setup:
   Remove the microtiter strips from the freezer and keep them at room temperature
Wash:
   Pipette 300*µ*l assay buffer into all wells and keep at RT for 1min. Therafter the buffer is decanted and discarded.
Assay incubation:
   Take the assay components out f the refrigerator and pipette sample (50*µ*l) first, tracer (50*µ*l) second and antibody (100*µ*l) third. Cover the plate with lid. Ioncubate 20h at 0-4°C in a dark and humid chamber.
Wash (4x):
   Empty the assay plate by decantation and add 300*µ*l cold wash solution to all wells. Repeat the procedure 3 times.
Enhancement:
   Empty the assay plate and pipette 200*µ*l enhancement solution to all wells and incubate for 60min at RT on a plate chaker in a dust-free chamber.
Measurement:
   Measure the time-resolved fluorescence (excitation: 340nm; emission: 615nm)

The FIA permitted measurements in the range from 0.04 to 9.72 pmol/ml (corresponding to 2 to 486 fmol/well). Within this range the intra- and inter-assay coefficient of variants of measured samples were generallly <15%. The slopefactore of the calibration curve was -1.07 and the EC50 was 0.32pmol. The specificity of the assay was determined by measuring cross-reactivity with structurally analogous compounds (e.g. cGMP, 2'3' cAMP, and other common nucleotides). Cross-reactivity for all tested compounds was < 0.0003%.

### Effect of selected PDE inhibitors on relaxin-stimulated cAMP production by human endometrial stromal cells.

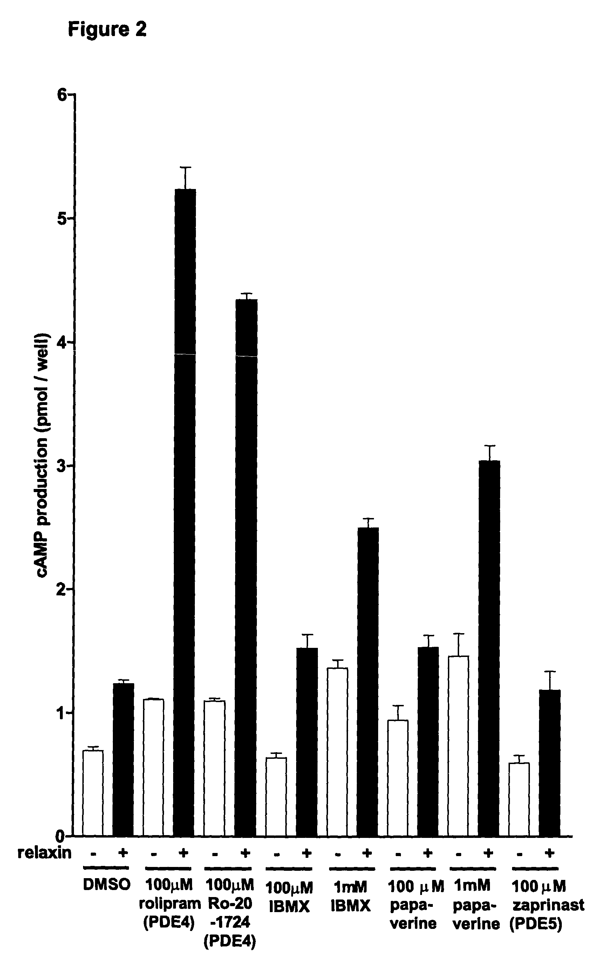

The peptide hormone relaxin is specifically able to induce cAMP production in cultured endometrial stromal cells, presumably acting through the newly discovered 7TM-receptor, LGR7 [Hsu et al., 2002, supra]. This effect of relaxin can be moderately increased by addition of either IBMX or papaverine at high concentrations (100*µ*M and 1mM) (Fig. 2). However, the basal level of cAMP is, as might be expected, also increased, such that the relative effect of relaxin is not changed (ca. 1.5 - 2-fold). Addition of the PDE4-specific inhibitors rolipram or Ro-20-1724, now at 100*µ*M (Fig. 2), results in a dramatic increase in cAMP production upon relaxin stimulation, which is supraproportional (ca. 4 - 5-fold; for some batches of cells (not shown) this may be as much as 20 - 25-fold). This result strongly suggests that one of the isoforms of PDE4, more than others, is specifically involved in relaxin-induced production of cAMP in human endometrial stromal cells.

### Effect of the protein kinase A inhibitor, H89, on PDE4 activity.

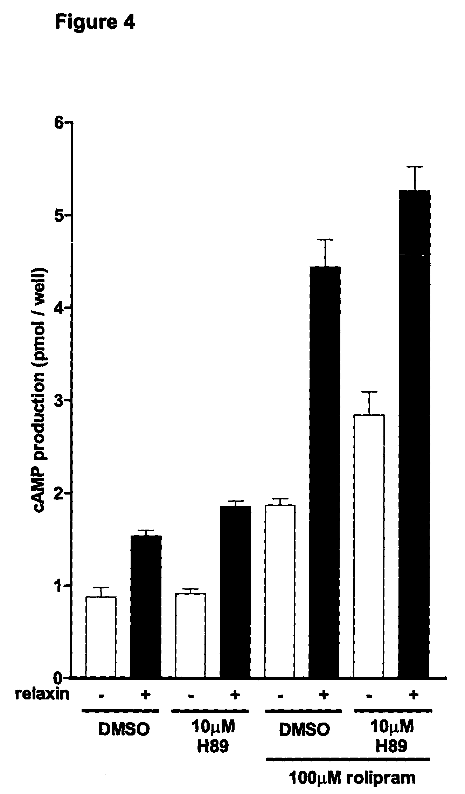

The short term role of cAMP to activate PDE, thereby reducing high intracellular cAMP concentrations within minutes of agonist binding to a receptor is generally accepted. Elevated cAMP concentration activates protein kinase A (PKA), which itself can activate several PDE4 subtypes by site-specific phosphorylation [Sette et al., 1994, J Biol Chem 269:9245-9252; and 1996, J Chem 271: 16526-16534; Ekholm et al., 1997, Biochim Biophys Acta 1356:64-70; Baillie et al., 2001, Mol Pharmacol 60:1100-1111]. As a further verification of such a pathway, the specific PKA-inhibitor H89 was applied to endometrial stromal cells in the presence and absence of the effector relaxin, and the PDE4-inhibitor rolipram (Fig. 4). As expected, both relaxin and rolipram, individually and in synergy together, caused a significant induction of cAMP. H89 caused a small but significant further increase in the cAMP generated in the presence of relaxin, with or without the PDE-inhibitor rolipram. This confirms just a small PKA-dependent stimulatory effect on a PDE, suggesting that a short-term PKA-dependent feedback inhibition of cAMP levels by activation of PDEs plays only a marginal role in ESC cells consistent with the sustained caMO response that is characteristic of decidualization.

### cAMP PDE Assay

Given the evidence of a role for PDE4 in human endometrial stromal cells, it is logical to determine whether longer term (3 days) application of a PDE4 inhibitor (rolipram) has the same physiological effect as specifically increasing intracellular cAMP. Transcript levels for the prolactin and IGF-BP1 genes, as markers of decidualization, were measured using semi-quantitative RT-PCR.

For this purpose confluent cultures of ESC in 92X17 TC dishes (Nunc) were washed twice in cold PBS, and cells harvested by scraping in ice-cold PBS. After centrifigation (200g, 4 min) cell pellets were resuspended in homogenization buffer (10mM Hepes (pH 7.4), 1mM EDTA, 1mM DTT) supplemented with a protease inhibitor cocktail (complete™, Roche Biochemicals, Mannheim, Germany). Cell suspensions were allowed to swell on ice for 8 min and then Dounce homogenized with 30 strokes. After addition of sucrose to a final concentration of 0.25M, the cell lysate was centrifuged at 1600g for 10min at 4°C. The supernatant was then frozen and stored immediately at -80°C until use. Protein concentration was determined using the Bio-Rad (Munich, Germany) kit, with bovine serum albumin (fraction V, Sigma) as standard. PDE activity was determined according to Stringfield & Morimoto (1997) with modifications. Extracts of ESC (0.6mg/ml protein, final concentration) were incubated at 30°C in a final concentration of 20mM Hepes (pH 7.4), containing 90mM KCl, 5mM MgCl₂, 0.75mM CaCl₂ and 1*µ*M cAMP, in the presence or absence 10*µ*M of the PDE inhibitors: IBMX, papaverine, rolipram, Ro-20-1724, dipyridamole, zaprinast, 8-methoxy-IBMX, EHNA (erythro-9(2-hydroxy-3-nonyl)adenine), cilostamide. All PDE inhibitors were purchased from Calbiochem, except papaverine (Sigma), and stocks dissolved in DMSO prior to further dilution and application. The diluted DMSO (final concentration 0.001%) was shown to have no effect on PDE activity. Reactions were terminated after 60min by acidifying with HClO₄ (final concentration 0.4M). After neutralization with 1/6 volume of saturated KHCO₃ solution, cAMP concentrations were measured by FIA on acetylated and diluted samples.

### Effect of various PDE inhibitors on the PDE activity in extracts of human endometrial stromal cells.

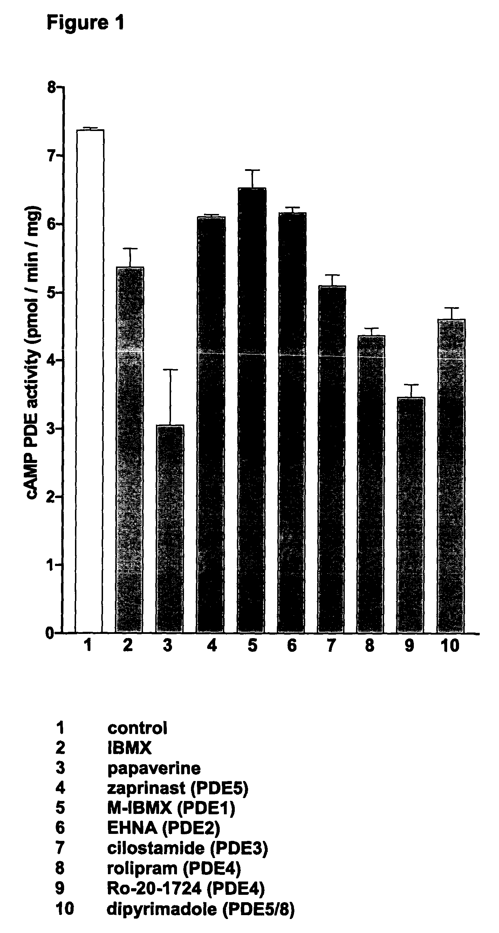

In an initial set of experiments, endogenous PDE activity was measured in endometrial stromal cell extracts, using a standard cAMP PDE assay, testing various PDE inhibitors for their ability to influence cAMP metabolism. Figure 1 illustrates experiments carried out with 10*µ*M of the inhibitors. This concentration was chosen to represent an amount close to that needed to completely inhibit the specific PDE, without, however, crossreacting with other PDEs (specific EC₅₀ values generally <5*µ*M; cross-reactivity >100*µ*M [Soderling et al., 1998, Acad Sci U S A 95:8991-8996]). Higher concentrations (100*µ*M) were also used (not shown), but while showing generally increased inhibition, there also appeared to be less PDE specificity. IBMX (inhibits most PDEs, except PDE8) proved moderately inhibitory in all experiments. Papaverine (inhibits most PDEs,-including PDE8) was more inhibitory, as were the PDE4 inhibtors rolipram and Ro-20-1724. Dipyridamole, which inhibits both PDE5 and PDE8, also showed significant inhibition. Since the specific PDE5 inhibitor zaprinast was without marked effect, dipyridamole is probably acting at a PDE8 type of phosphodiesterase. In repeated experiments, the specific inhibitors of PDE1, PDE2, PDE3 and PDE5 showed no or only small effects. Taken together, these results suggest that both PDE4 and PDE8 are the more important PDE isoforms involved in CAMP catabolism in human endometrial stromal cells.

### Example 3

### Identification of the relevant PDE

Both PDE4 and PDE8 activity can result from expression of different genetic isoforms. PDE4 activity is encoded by four related genes (PDE4A, -4B, -4C and -4D) that can encode numerous isoforms through alternate splicing of RNA [Houslay et al., 1998, supra]. PDE8 activity is encoded by two closely homologous genes PDE8A and PDE8B [Fisher et al., 1998, Biochem Biophys Res Commun 246:570-577; Hayashi et al., 1998, Biochem Biophys Res Commun 250:751-756]. The relevant PDEs were identified by RT-PCR.

### RT-PCR Analysis

Total RNA was extracted from ESC of each experimental condition using RNeasy kits (Qiagen, Hilden, Germany) according to the manufacturer's instructions. cDNA was generated from 5*µ*g total RNA using 0.5*µ*g oligo[dT] primer (Invitrogen, Karlsruhe, Germany) in a total volume of 12*µ*l. After denaturing at 70°C for 10min with immediate cooling, the solution was supplemented with first strand buffer (Invitrogen), 20mM (end concentration) of each dNTP (Genecraft, Münster, Germany), 0.1M DTT (Invitrogen) and preincubated for 2min at 42°C. This was followed by 200U Superscript II reverse transcriptase (Invitrogen) to give a total volume of 20µl, and incubation continued for a further 30min. Reactions were stopped by heating at 70°C for 10min and were stored at -20°C. Preparations without reverse transcriptase were used as negative controls, wherein the absence of PCR products indicated a complete lack of contaminating genomic DNA.

PCR reactions were performed in a 50*µ*l total reaction volume, containing 1*µ*l of cDNA, 200nM each sense- and antisense-primer, 0.1U BioTherm-Red™ DNA polymerase (Genecraft), 200*µ*M of each dNTP in 1x buffer (Biotherm™; Genecraft). Primers for each PDE4 subtype (Table 1) were those used by Leroy et al. (1999, Cell Signal 11:31-37) (PDE4A & C) and Erdogan and Houslay (1997, Biochem J 321:165-175) (PDE4B & D). Primers for hPRL and IGF-BP1 transcripts were as in Brosens et al. [1996, Endocrinology 137: 2225-2231], and for GPDH as previously described [ref] (Table 1). Before amplification, samples were denatured at 95°C for 5min. The amplification profile for each specific PCR reaction, consisting of denaturation at 94°C, annealing at the specific temperature and extension at 72°C, as given in Table 2, followed by a final extension for 10min at 72°C. The identity of all PCR products was confirmed by DNA sequencing using the dideoxy termination procedure. A 20*µ*l aliquot of each reaction mixture was electrophoresed on a 1% agarose gel and visualized by ethidium bromide fluorescence using the ImaGo™ system (B&L Systems, Maarssen, Netherlands). Band intensities were analysed densitrometrically using the Image Quant 5.0 software package (Molecular Dynamics), and results expressed as percent of untreated control (mean +/- s.e.; n = 4).

### RT-PCR identification of PDE4 and PDE8 isoforms in human endometrial stromal cells.

Transcript-specific PCR assays were developed using specific oligonucleotides capable of discriminating between PDE4A, -4B, -4C and -4D, as well as the two PDE8 isoforms.

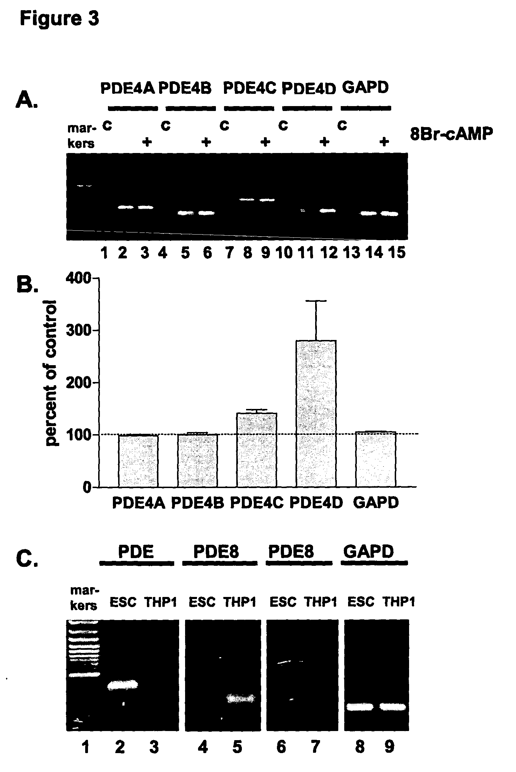

In Figure 3 (panel A), it can be seen that all PDE4 isoforms are indeed expressed in human endometrial stromal cells. Interestingly, treatment of the cells for 3 days with 8Br-cAMP has no influence on PDE4A or PDE4B transcripts, but significantly upregulates gene expression for PDE4C and PDE4D. In fact, PDE4D transcript levels are already significantly upregulated after only 4 h of 8Br-cAMP treatment (not shown). PDE8B is the major isoform expressed in endometrial stromal cells (Fig. 3B). PDE8A transcripts can be detected eventually after a second full round of PCR (not shown). In contrast, in the THP-1 cell line which also responds to relaxin to produce an upregulation of cAMP and is used here as control, PDE8B appears to be absent; instead PDE8A is highly expressed. Treatment of the endometrial stromal cells with 8Br-cAMP had no effect on the transcript level of PDE8B (not shown). The identity of all PCR products was verified by DNA sequencing. The effects observed are thus clearly mediated by PDE-4 activity.

### Example 4

### Decidualization of human Endometrial Stromal Cells

Given the evidence of a role for PDE4 in human endometrial stromal cells, it was determined whether longer term (3 days) application of a PDE4 inhibitor (rolipram) has the same physiological effect as specifically increasing intracellular cAMP.

### hPRL ELISA

ESC cells in 6-well plates were incubated with combinations of relaxin, progesterone and different inhibitors of phosphodiesterase as described above. For the determination of hPRL release, 1ml conditioned ESC medium was stored at -80°C until use, and hPRL content later determined in the undiluted medium using a conventional ELISA format. This employed a hPRL-specific rabbit polyclonal antibody, previously validated for radioimmunoassay (Gellersen et al., 1989, Endocrinology 125:2853-2861), with a biotinylated hPRL-tracer and horseradish peroxidase-conjugated streptavidin as detection system. hPRL standards (prepared in the laboratory of Dr. H.G. Friesen, University of Manitoba, Canada) were dissolved in basal ESC culture medium (as above) plus 0.001% DMSO. Binding capacity of the ELISA in conditioned medium was ca. 93% of values determined in standard E-PBS (0.1M sodium phophate, pH7.0; 0, .15M NaCl; 5mM EDTA; 0.2% bovine serum albumin; 0.01% thimerosal). The preparation of the biotinylated hPRL-tracer, as well as the complete assay design, followed the procedure described for a relaxin-ELISA (Einspanier et al., 1999, Biol Reprod, 61:512-520). Sensitivity was in the range 0.5 to 81ng/ml (equivalent to 25 to 4050 pg/well). The slopefactore of the calibration curve was -1.16 and the EC50 was 5.06 ng hPRL/ml. Intra- and inter-assay coefficients of variants were 6.2% and 9.7%, respectively.

The following materials are used for the hPRL-ELISA:
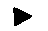 Immuno modules:
   Nunc 469949, coated with goat anti-rabbit-gamma-globulin
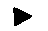 Lid:
   (Nunc 460348)
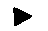 Assay buffer (EPBS):
   0.10M sodium phosphate
   0.15M NaCl
   0.005M EDTA
   0.20% BSA
   0.01% Thimerosal
   pH 7.0
> hPRL-antiserum:
   Affinity purified antiserum (MS1 rabbit). Pool 6-25, dilution 1:70.000 in EPBS-buffer + 0.0005% methylorange
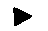 hPRL-tracer :
   lyophilized aliquots of biotinylated hPRL
   ready-to-use : 12ng / ml in EPBS + 0.0005% bromphenylblue
   dissolve lyophilizate in tracer buffer
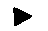 Wash solution:
   0.02% Tween20 in H2O
   0.5% NaCl
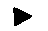 Horseradishperoxidase (HRP)-Streptavidin solution:
   150ng / ml cold EPBS
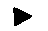 HRP-substrate solution:
   25ml H20
   500*µ*l substrate buffer
   500*µ*l 0.02%H202
   500*µ*l 0.5% TMB (3,3',5,5'-Tetramethylbenzidin) in DMSO
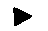 Substrate buffer:
   4.80M Na-Acetat
   0.24M Citronensäure

### Working protokoll for hPRL-ELISA:

### Plate setup:

Remove the microtiter strips from the freezer and keep them at room temperature

### Wash Pipette:

375*µ*l assay buffer into all wells and keep at RT for 2min. Therafter the buffer is decanted and discarded.

### Assay incubation:

Take the assay components out of the refrigerator and pipette sample (50µl) first and second antibody (100µl). Cover the plate with lid. Incubate 18-24hrs at 4-6°C in a dark and humid chamber. After this first incubation add 50*µ*l of tracer solution and incubate another 2hrs at 4-6°C.

### HRP-Strept.-Incubation:

Decant wells and 0.2ml HRP-Streptavidin solution per well. Incubate 30min at 4-6°C in the dark.

### Wash (4x):

Emty the assay plate by decantation and add 375*µ*l cold wash solution to all wells. Repeat the procedure 3 times.

### Substrate incubation:

Empty the assay plate and pipette 250*µ*l substrate solution to all wells and incubate for 40min at RT in a dark chamber.

### Stop:

Pipette 50*µ*l H2SO4 to all wells

### Measurement:

Measure the OD at 450nm

### RT-PCR Analysis

RT-PCR was carried out essentially as outlined above (Example 3).

### Application of PDE4 inhibitors to induce decidualizaton in human endometrial stromal cells.

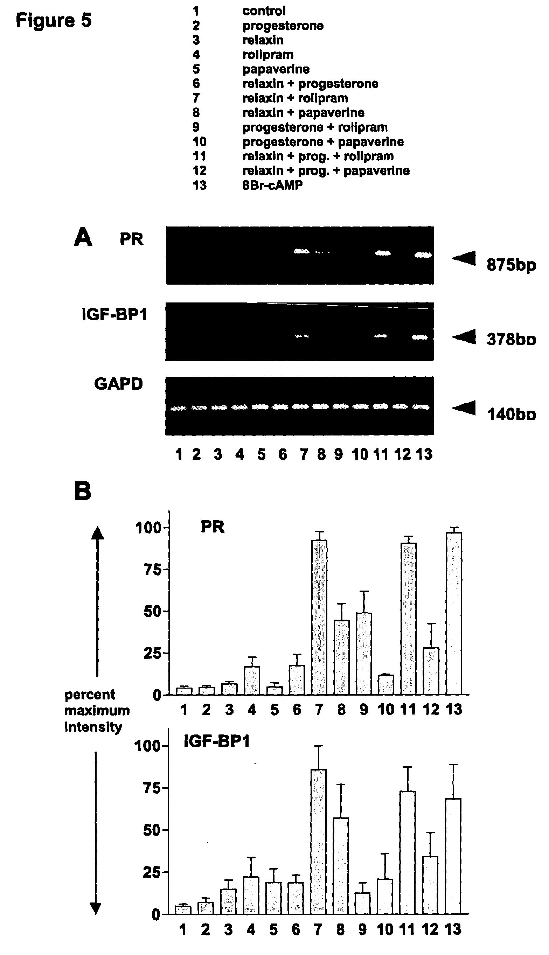

Transcript levels for the prolactin and IGF-BP1 genes, as markers of decidualization, were measured using semi-quantitative RT-PCR. Four completely independent batches of primary cells were assessed and significance estimated by ANOVA (Fig. 5). Prolactin mRNA can be induced by rolipram alone to a greater extent than by the hormone effector relaxin alone. Together, there is marked synergy of these two factors. Essentially similar results were observed also for the mRNA for IGF-BP1, though the levels of transcript detected when relaxin or rolipram were added alone were very low. The synergy between both factors was nevertheless highly significant. Of interest here, is that progesterone has no detectable effect, either alone, or in conjunction with relaxin. Only when applied together with rolipram is a significant effect evident, and then only for prolactin mRNA but not for IGF-BP1 mRNA. Papaverine a more general PDE inhibitor was also effective, though not to the same extent as rolipram. Similar experiments were also carried out for up to 6 days (not shown) with very similar results.

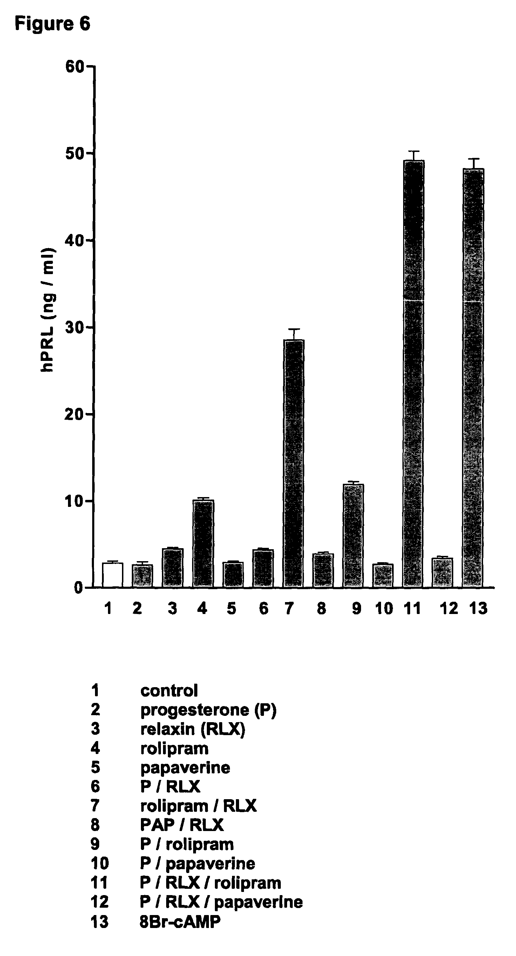

To confirm the decidualization phenotype using a different parameter, also prolactin peptide secretion into the culture medium was measured (Fig. 6). The results were very similar to those reporting specific gene transcript levels, with the PDE4-inhibitor rolipram being effective alone, but showing marked synergy in conjunction with relaxin, and progesterone being without any effect. In this system, papaverine had a smaller, though still significant effect, compared to rolipram. It should be noted, that the combination of relaxin and rolipram, is almost as effective as giving pure 8Br-cAMP. In these assays, relaxin alone or with progesterone was unable to increase the amount of prolactin detectable in the culture medium.

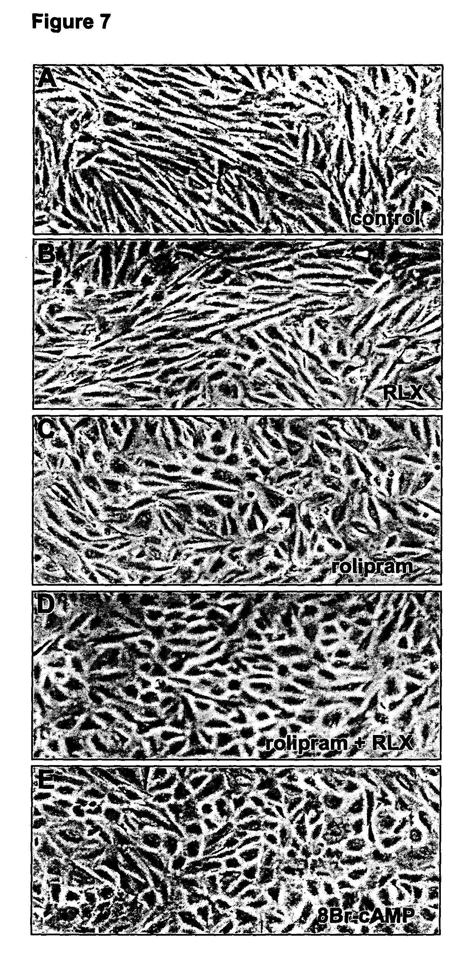

Finally, the cells in culture were checked for the morphological changes associated with decidualization, as described in Christian et al., Reprod Biomed Online, 3:24-30, 2002 (see Fig. 7). Here, cells were photographed on day 2, when the differences in differentiation rate between treatments was most apparent. In terms of the proportion of cells showing the typical cobblestone pattern of decidualized endometrial stromal cells, the various substances were effective in the order: vehicle < relaxin < rolipram < relaxin+rolipram < 8Br-cAMP.

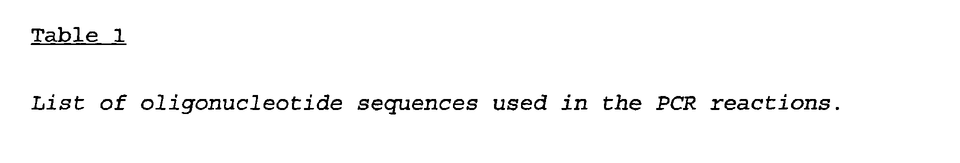

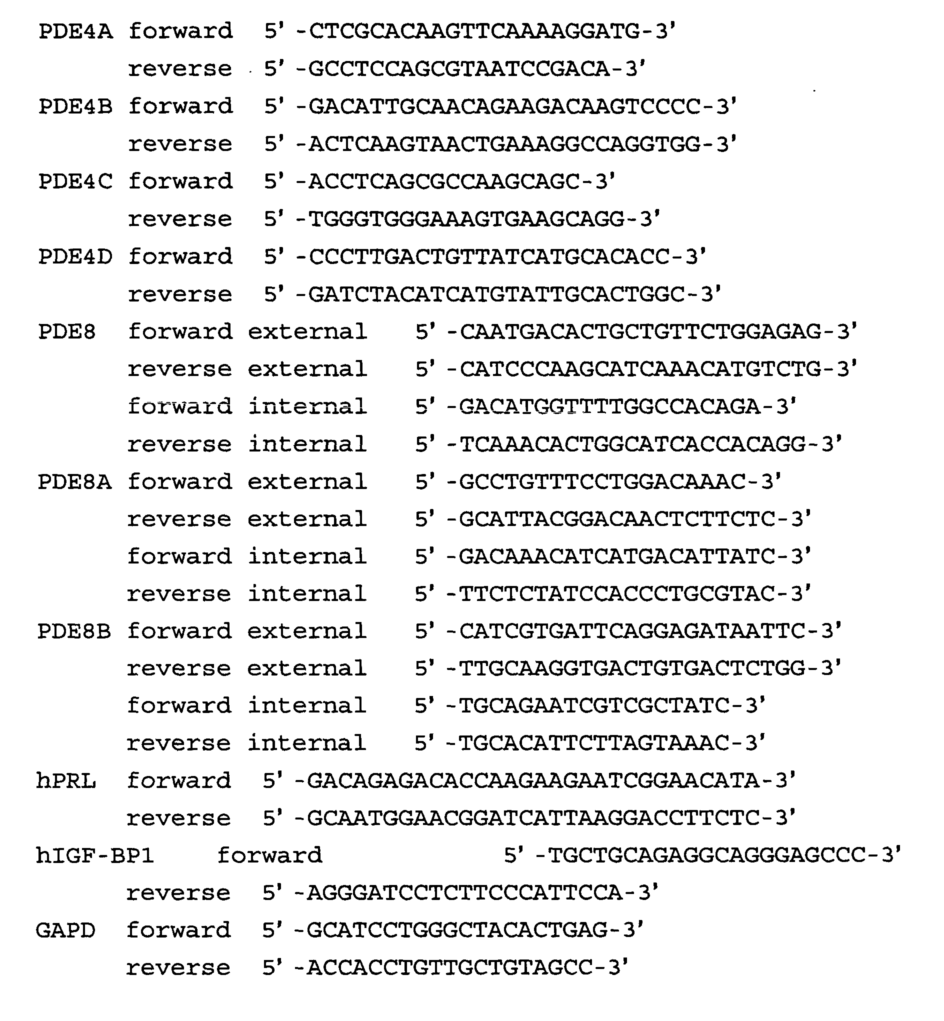

**Table 2**

| *Amplification* | *conditions for the PCR reactions.* |
|---|---|
| PDE4A,B,D | 35 cycles: 94°C 1 min / 56°C 1 min / 72°C 90 sec |
| PDE4C | 38 cycles: 94°C 1 min / 64°C 1 min / 72°C 1 min |
| PDE8, 8A&B | 30 cycles: 94°C 45 sec / 56°C 30 sec / 72°C 1 min |
| PDE8, 8A&B (nested) | 30 cycles: 94°C 30 sec / 47°C 30 sec /72°C 1 min |
| hPRL | 30 cycles: 94°C 30 sec / 50°C 30 sec / 72°C 1 min |
| hIGF-BP1 | 30 cycles: 94°C 45 sec / 50°C 1 min / 72°C 1 min |
| GAPD | 30 cycles: 94°C 30 sec / 55°C 30 sec / 72°C 1 min |

## Claims

1. Use of an inhibitor, which inhibits a specific type of cAMP specific phosphodiesterase-4 enzyme (PDE4), for the preparation of a pharmaceutical composition for promoting the decidualization of endometrial cells.

2. Use according to claim 1, wherein the inhibitor has an inhibiting effect on one or several of the isoforms of PDE4, such as PDE4-A, -B, -C and/or -D.

3. Use according to claim 1 or 2, wherein inhibitor is rolipram (Schering/ Calbiochem), Ro-20-1724 (Calbiochem), Arophyline (Allmiral), Cilomilast (GlaxoSmithKline), Roflumilast (Byk Gulden), V-11294A (Napp), CDC-801 (Celgene), Bay-19-8004 (Bayer), Cipamphylline (SmithKlineBeecham), SCH-351591 (Schering Plough), D-4418 (Schering Plough), YM-976 (Yamanouchi), PD-189659 (Pfizer), CI-1018.9 (Park Davis), Mesopram (Schering AG), Pumafentrine (Byk Gulden), CDC-998 (Celgene), IC-485 (Icos), KW-4490 (Kyowa), RPR-73401, RPR-109026, L-791,943 (Merck), CDP-840 (Celltech), CI-1044, XT-44 or pentoxyfylline.

4. Use according to one of claims 1 to 3, wherein the pharmaceutical composition further comprises a compound which increases the synthesis of cAMP.

5. Use according to one of claims 1 to 4, wherein the compound which increases the synthesis of cAMP is relaxin or an agonist of the action of relaxin receptors such as relaxin receptors LGR7 and/or LGR8.

6. Use according to one of claims 1 to 5, wherein the agonist is a peptide analogue.

7. Use according to one of claims 1 to 5, wherein the agonist is a non-peptide mimetic.

8. Use according to one of claims 1 to 7, wherein the pharmaceutical composition is used for the treatment of humans, specifically for the treatment of women.

9. Use according to one of claims 1 to 8, wherein promoting the decidualization of endometrial cells increases the blastocyst implantation rate.

10. Use according to one of claims 1 to 9, wherein promoting the decidualization of endometrial cells increases the rate of pregnancy.

11. Use according to one of claims 1 to 10, wherein the pharmaceutical compostion further comprises pharmaceutically acceptable carrier and/or diluents.

12. Use according to one of claims 1 to 11, wherein the pharmaceutical composition is administered before and/or during before and/or after implantation of the blastocyst.

13. Use according to one of claims 1 to 12, wherein the pharmaceutical composition is administered before and/or after implantation of the blastocyst obtained by artificial fertilization.

14. Use according to one of claims 1 to 13, wherein the artificial fertilization is/are in vitro-fertilization.

15. Use according to one of claims 1 to 13, wherein the pharmaceutical composition is used for the treatment of endometrial disorders, such as endometriosis, adenomyosis, luteal phase defects and menstrual disorders.

16. Use according to one of claims 1 to 15, wherein the pharmaceutical composition is formulated for oral, intravenous or vaginal administration.

17. Use according to one of claims 1 to 16, wherein the pharmaceutical composition comprising the PDE4 specific inhibitor alone or in combination with relaxin or an agonist of the action of relaxin receptors is administered repeatedly over a period of at least 3 days, preferably over a period of at least 2 weeks.

18. Use according to one of claims 1 to 17, wherein the composition is administered dayly or several times per day.

19. Use according to one of claims 1 to 18, wherein the composition comprises the PDE4 specific inhibitor in combination with relaxin and is administered over a period of at least 3 days several times a day to women before and/or after implantation of the blastocyst.

20. Pharmaceutical composition comprising an inhibitor, which inhibits a specific type of cAMP specific phosphodiesterase-4 enzyme (PDE4), and relaxin or an agonist of the action of relaxin receptors.

21. Pharmaceutical composition according to claim 20, wherein the compound which increases the synthesis of cAMP is relaxin or an agonist of the action of relaxin receptors such as relaxin receptors LGR7 and/or LGR8.

22. Pharmaceutical composition according to claim 20 or 21, whereinthe agonist is a peptide analogue.

23. Pharmaceutical composition according to claim 20 or 21, wherein the agonist is a non-peptide mimetic.

24. Pharmaceutical composition according to one of claims 20 to 23, wherein the inhibitor inhibits one or several of the PDE isoforms of the PDE type, such as PDE4-A, -B, -C and/or -D.

25. Pharmaceutical composition according to one of claims 20 to 24, wherein inhibitor is rolipram (Schering/ Calbiochem), Ro-20-1724 (Calbiochem), Arophyline (Allmiral), Cilomilast (GlaxoSmithKline), Roflumilast (Byk Gulden), V-11294A (Napp), CDC-801 (Celgene), Bay-19-8004 (Bayer), Cipamphylline (SmithKlineBeecham), SCH-351591 (Schering Plough), D-4418 (Schering Plough), YM-976 (Yamanouchi), PD-189659 (Pfizer), CI-1018.9 (Park Davis), Mesopram (Schering AG), Pumafentrine (Byk Gulden), CDC-998 (Celgene), IC-485 (Icos), KW-4490 (Kyowa), RPR-73401, RPR-109026, L-791,943 (Merck), CDP-840 (Celltech), CI-1044, XT-44 or pentoxyfylline.
